# EUROPEAN PATENT APPLICATION

(11) **EP 3 766 572 A1**
(43) Date of publication of application: **20.01.2021**
(21) Application number: 20186365.1
(22) Date of filing: 17.07.2020
(51) Int. Cl.: B01J 19/12, A61K 9/20

(54) **AN APPARATUS AND A METHOD FOR PRODUCING A CHEMICAL PRODUCT**

(30) Priority: 17.07.2019 LU 101313
(71) Applicant: Univerza V Ljubljani, 1000 Ljubljana (SI)
(72) Inventor: PLANINSEK, Odon, 1000 Ljubljana (SI)
(74) Representative: Zacco GmbH

(57) **Abstract**

A method for producing a chemical product, in particular a pharmaceutical drug, comprises the steps of
providing a powder (23) comprising a porous first excipient and a substance, the substance comprising at least one component having a melting point which is below the melting point of the first excipient, and
producing the chemical product by heating the powder (23), in particular by use of an infrared light source, to a process temperature between the melting point of the component and the melting point of the first excipient.

## Description

The present disclosure relates to an apparatus and a method for producing a chemical product, in particular a pharmaceutical drug.

For pharmaceutical purposes, solid dispersions are mainly produced in order to provide a relatively rapid dissolution in water for poorly soluble active pharmaceutical ingredients. Solid dispersions were introduced by Sekiguchi and Obi in 1961, see in the scientfic publication of K. Sekiguchi, N. Obi. Studies on absorptions of eutectic mixture. I. A Comparison of the Behavior of Eutectic Mixture of Sulfathiazole and that of Ordinary Sulfathiazole in Man. Pharm Bull, 9 (1961), pp. 866-872.

Such solid dispersions can, for example, comprise an active pharmaceutical ingredient, which is poorly soluble in water, and an excipient which is, for example, a solid dispersion former and which is typically well soluble in water. Solid dispersions can be manufactured also with excipients, which are water nonsoluble. Such excipients are mainly porous or mesoporous materials with a diameter of pores between 2 nm and 50 nm. Mesoporous materials have according to the International Union of Pure and Applied Chemistry (IUPAC) nomenclature pores between 2 nm and 50 nm. Materials that can be used for this purpose are porous silicon dioxide, porous silicon, porous calcium carbonate, magnesium aluminum silicate (type Neusilin), porous ceramics, porous calcium silicate, and polypropylene foam in the form of powder (type Accurel).

In the use of such excipients, pores can be impregnated with an active pharmaceutical ingredient to form a solid dispersion. An improved dissolution can be a result of the wettability improvement, of an increase in specific surface area and/or of an amorphous state of the active pharmaceutical ingredient. Porous excipients or carriers can be impregnated with the use of a solution of the active pharmaceutical ingredient in the solvent, which passes into the pores. Disadvantages of using solutions of organic solvents might be: toxicity, price and a negative impact on the environment.

In a mechanochemical method for the manufacture of solid dispersions, where a grinding step is employed can be problematic from the perspective of a continuous production. A device by which solid dispersions can be manufactured is described in US 3 410 938 A.

When solid dispersions are produced by melt extrusion with use of a porous excipient and an active pharmaceutical ingredient, a further component such as hydrophilic organic polymer can be added, for example hipromelose, copovidone or polyvinyl alcohol poliethileneglykol crosslinked copolymer, see in the scientific publication of Hanada M., Jermain St., Williams RO III, Enhanced Dissolution of a Porous Carrier-Containing Ternary Amorphous Solid Dispersion System Prepared by a Hot Melt Method, Journal of Pharmaceutical Sciences 2018 Jan; 107(1): 362-371. The share of the active pharmaceutical ingredient in the formulation at the end may be relatively low as it is for the achievement of the relevant flow properties of the particles necessary to add other excipients (fillers, glidants).

Known methods for the melting of the active pharmaceutical ingredient and its penetration into the pores of porous excipients comprise heating with hot air - see in the scientific publication of Robert J. Ahern, John P. Hanrahan, Joseph M. Tobin, Katie B. Ryan, Abina M. Crean, Comparison of fenofibrate-mesoporous silica drug-loading processes for enhanced drug delivery, European Journal of Pharmaceutical Sciences, Volume 50, Issues 3-4, 20 November 2013, Pages 400-409 - or the use of microwaves - see in the scientific publication of L. J. Waters, T. Hussain, G. Parkes, et al., Inclusion of fenofibrate and a series of mesoporous silicas using microwave irradiation, European Journal of Pharmaceutics and Biopharmaceutics 85 (2013) 936-941.

It is an objective of the present invention to provide an apparatus and a method which can be employed to produce in an improved way a chemical product, in particular a product that is a solid dispersion during its production process or as an end product.

The objective is satisfied by a method in accordance with the features of claim 1, an apparatus in accordance with the features of claim 8, and an apparatus in accordance with the features of claim 10. Preferred embodiments of the present invention are described in the dependent claims.

In accordance with some embodiments of the present invention, an apparatus for producing a chemical product, in particular a pharmaceutical drug, comprises:
a heater for generating the chemical product by heating a powder when the powder is arranged in the heater, the powder comprising a porous first excipient and a substance, the substance comprising at least one component having a melting point which is below the melting point of the first excipient,
wherein the heater comprises an infrared light source which is configured to heat the powder to a process temperature between the melting point of the component and the melting point of the first excipient.

In particular, the apparatus is suitable or intended for continuously producing the chemical product. The apparatus can comprise a transport device that enables or contributes to enable the continuous process.

The infrared light source allows the powder to be heated to the process temperature which is above the melting point of the component of the substance. During heating, the component melts and can therefore penetrate into the pores of the first excipient which does not melt, since the process temperature is below its melting point. Alternatively, the component might include very small, in particular nano-size, particles of the active pharmaceutical ingredient. These particles can penetrate into the pores while the melt does not penetrate into the pores.

The wording "when the powder is arranged in the heater" means in particular that the powder is arranged such that it is in the area of effect of the heater. This in particular means that the powder is exposed directly or indirectly to infrared light from the light source. The powder and the chemical product or components thereof are not part of the claimed apparatus. The area of effect of the heater can in particular be related to the area of a surface, on which the powder is arranged and that can be directly illuminated by the light source.

In the powder, the first excipient and the substance are preferably provided in powder form. Thus, each of the first excipient and the substance consists of a plurality of very small particles that together form the powder. Thus, the powder is preferably a granular material and as such a mixture of the particles of the first excipient and the substance. When heated to the process temperature, the melted particles of the component of the substance or small size particles, such as nanoparticles, in the melt can penetrate into the pores of the particles of the first excipient in the powder volume.

The substance might include at least a second component which does not melt when heated to the process temperature. However, as the second component also has a powder form and thus consists of a plurality of small particles, the particles of the second component can be transported by the melted first component towards the pores of the first excipient. In particular, the second component can be an active pharmaceutical ingredient having very small particles, in particular nanoparticles. These particles can penetrate into the pores. In contrast, the particles of the melted first component can be micro-size particles. Thus, they can have a size of a few tenth of micrometers. These particles can be bigger than the particles of the porous first excipient and they do therefore not penetrate into the pores.

The first component can be a second excipient while the second component can be an active pharmaceutical ingredient that, preferably, has a melting temperature that is above the process temperature. After heating, the resulting chemical product can be regarded as a solid dispersion in which the active pharamceutical ingredient has been brought into the pores of the first excipient by use of the melted second excipient. Due to the temporarilly melted second excipient, the particules that finally form the resulting chemical product can be »backed together«, for example, in tablet form. Alternatively, the resulting chemical product can be in powder form.

The use of an infrared light source to heat the powder is particularly advantageous because it allows the process temperature to be controlled very accurately. Furthermore, the process temperature can be reached rapidly after a switch on of the infrared light source and it can be kept constant over the illuminated surface area.

The apparatus can be employed for making solid dispersions of one or more active pharmaceutical ingredients and one or more porous first excipients with the use of an elevated process temperature, which is provided by the source of infrared light. The elevated process temperature causes the conversion of a pure solid active pharmaceutical ingredient or a combination of active pharmaceutical ingredients with meltable second excipients into melt and the penetration of the melt or of the active pharmaceutical ingredients into the pores of the porous nonmeltable first excipient.

The apparatus might comprise a transport device for transporting the powder in a transport direction to the heater and/or for transporting the chemical product in the transport direction away from the heater. Therefore, a continuous production process can be implemented. In particular, the powder can be transported with a constant velocity along the transport direction.

The transport device can be employed to transport the powder through the area of effect of the heater such that the powder is exposed to the infrared light from the infrared light source while transported along the transport direction. The power of the infrared light can be set such as to heat the powder to the process temperature while it is exposed to infrared light and to ensure that the substance or at least the particles of the active pharmaceutical ingredient as a component of the substance can penetrate into the pores of the first excipient. This can be done in such a way that the transport movement of the powder does not need to be stopped.

The transport device can comprise a conveyer belt serving as support surface for the powder and/or the chemical product. The conveyor belt can serve as a simple and robust means for transporting the powder and/or the chemical product.

In accordance with some embodiments of the invention, an apparatus for producing a chemical product comprises:
a heater for generating the chemical product by heating a powder when the powder is arranged in the area of effect of the heater, the powder comprising a porous first excipient and a substance, the substance comprising at least one component having a melting point which is below the melting point of the first excipient, and
a transport device which comprises a conveyer belt serving as support surface for the powder and/or the chemical product, the conveyor belt being configured to move the powder through the area of effect of the heater. This is particularly advantageous as the chemical product can be produced while the powder and the resulting chemical product are transported on the conveyor belt. A high production rate can thereby be achieved.

In some embodiments, the infrared light source is arranged such as to illuminate a portion of the conveyor belt. The powder on the portion of the conveyor belt can be heated by infrared light from the light source. As the conveyor belt moves, all areas of the support surface of the conveyor belt are gradually illuminated by the infrared light.

Preferably, the infrared light source is arranged in a housing of the heater. The housing can be open at its bottom side which faces the support surface of the conveyor belt. The powder can therefore be transported by the conveyor belt to the heater and the produced product can be transported away from the heater. The light source can be arranged in a top portion within the housing such that a light emitting surface of the light source is directed to the support surface of the conveyor belt. The area of effect of the heater therefore covers the illuminated portion of the support surface of the conveyor belt.

The heater can comprise at least one temperature sensor. The temperate provided by the infrared light source can therefore be monitored.

The heater can comprise two or more temperature sensors. The temperature sensors can be arranged at a distance from each other along the transport direction. Preferably, the temperature sensors are arranged within a housing of the heater. The temperature sensors allow the temperature to be measured at different locations along the transport direction. The temperature sensors can be employed to obtain a process temperature which is at least approximately constant at different locations along the transport direction and inside the housing.

The apparatus can comprise a dispensing device for providing the powder on a transport device, in particular on a conveyor belt, which is configured to transport the powder along a transport direction to the heater. The dispensing device can serve as an element for delivering a homogenous physical mixture of the first excipient and the substance in powder form on the conveyor belt. The dispensing device can provide repeatedly a defined amount of the powder on the conveyor belt. Thus, portions of the powder can be formed on the conveyor belt.

The apparatus might comprise a compaction device for compacting the powder. In particular, the portions provided by the dispensing device on the conveyor belt can be pressed and compacted by use of the compaction device.

The compaction device can comprise a compaction element arranged above the conveyor belt for transporting the powder from the dispensing device to the heater, and the compaction element can be movable towards the conveyor belt. The compaction element can in particular be configured to press a portion of the powder against the conveyor belt.

The compaction device might be arranged between the dispensing device for providing the powder on the transport device and the heater. Thus, a powder portion can be compacted while it is lying on the conveyor belt and before it is provided to the heater.

The apparatus can comprise a cooler for cooling the chemical product after removal from the heater. The conveyor belt might be configured to transport the chemical product through the area of effect of the cooler.

The cooler can comprise a cooling element which can be arranged in a housing of the cooler. The housing can be open at its bottom side which faces the support surface of the conveyor belt. The chemical product can therefore be transported by the conveyor belt through the area of effect of the cooler. The cooling element can be arranged in a top portion within the housing and above the support surface of the conveyor belt.

A transport device might be configured to transport the chemical product from the heater to the cooler and/or for removing the chemical product from the cooler. The transport device can be used to implement a continuous production process, in particular by using a conveyor belt which is operated at a constant transport velocity.

The invention also relates to a method for producing a chemical product, in particular a pharmaceutical drug, the method comprising the steps of
providing a powder comprising a porous first excipient and a substance, the substance comprising at least one component having a melting point which is below the melting point of the first excipient,
producing the chemical product by heating the powder to a process temperature between the melting point of the component and the melting point of the first excipient.

In accordance with some embodiments, the method enables the production of solid dispersions which consist of or comprise one or more active pharmaceutical ingredients and one or more porous excipients, in particular by using a production apparatus that comprises a part which allows the active pharmaceutical ingredients and excipients to be heated to form a melt of active pharmaceutical ingredients or a melt of active pharmaceutical ingredients in a mixture with a melted excipient such that the active pharmaceutical ingredients can penentrate into the pores of a porous excipient which does not melt at the used process temperature. The one or more excipients that melt in combination with one or more active pharmaceutical ingredients which are used in the production of the solid dispersion can be added in low proportion and might allow reducing the melting point of the active pharmaceutical ingredient to preserve its stability. Lowering the melting point can also help to reduce the energy consumption.

The substance can comprise or consist of an active pharmaceutical ingredient.

The substance might comprise an active pharmaceutical ingredient and a second excipient, the second excipient being the component having the melting point which is below the melting point of the first excipient.

In some embodiments, the powder is heated to the process temperature until the particles of the active pharmaceutical ingredient have penetrated into the pores of the first excipient.

The heating step can comprise the step of exposing the powder to infrared light.

The heating step can comprise the step of moving the powder along a transport direction, in particular while exposing the powder to infrared light.

In some embodiments, an apparatus can comprise or consists of an element which can deliver a homogeneous physical mixture of powders of an active pharmaceutical ingredient and, if neccesary, of a meltable excipient and the porous nonmeltable excipient to a transport element, such as a conveyor belt. The transport element can pass this mixture, for example, in the form of an evenly distributed layer of the particles or in the form of a slight compact, which provides good contact between the particles and the effective formation of the dispersion with reproducible properties, to an infrared heater. The heater can melt a pure active pharmeceutical ingredient or its mixture with a meltable excipient that possesses a low melting point in combination with a porous nonmeltable excipient. A solid dispersion is then formed after heating with melt impregnation and cooling.

In some embodiments, the method comprises a step of compacting the powder. This step can be performed by use of a compaction device. The compaction device can interact with the conveyor belt to compact the powder which is placed on the conveyor belt below the compaction device.

Compacting the powder might assure an improved contact between particles of the powder and thus enable or improve pores impregnation with melted materials during heating.

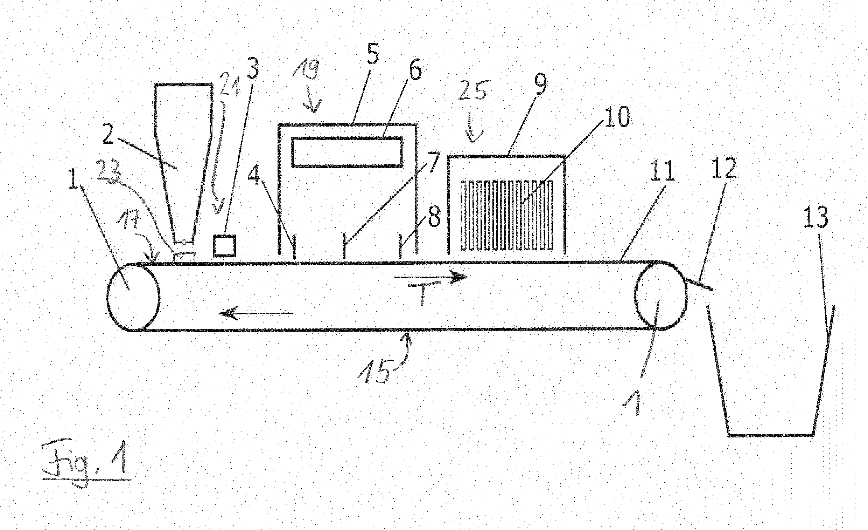

Examplary embodiments of the present invention will now be described with reference to Fig. 1 which shows schematically a side view of an examplanary apparatus for producing a chemical product.

The apparatus shown in Fig. 1 comprises a transport device 15 which comprises a conveyor belt 11 which is movable in a transport direction T. The conveyor belt revolves around rotating rollers 1. Several components of the apparatus are arranged along the transport direction T above the conveyor belt 11.

The apparatus includes a dispensing device 2 that is configured to provide portions 23 of a powder on the support surface 17 of the conveyor belt 11. The dispensing device 2 therefore can serve for the delivery of a homogenous physical mixture of portions 23 of powder onto the conveyor belt 11.

A portion 23 of the powder consists of or comprises particles of a porous first excipient and particles of a substance, which comprises at least one component, in particular an active pharmaceutical ingredient or a mixture of an active pharmaceutical ingredient and a second excipient. The substance has a melting temperature which is below or equal to a process temperature provided by a heater 19.

The apparatus comprises between the dispensing device 2 and the heater 19 a compaction device 21 which comprises a compaction element 3 that is arranged above the support surface 17 of the conveyor belt 11 and that is movable towards the conveyor belt 11. A powder portion 23 placed by the dispensing device 2 on the support surface 17 can be compressed and compacted by the compaction element 3 after it has been transported by the conveyor belt 11 such that it is located below the compaction element 3.

The compacted portion of powder 23 can be further transported to the heater 19. As shown in Fig. 1, the heater 19 comprises a housing or heating compartment 5 which is arranged above the conveyor belt 11 and which is open at its bottom side. An infrared light source 6 is arranged within the compartment 5 such that its light emitting surface is facing downwards to illuminate the portion of the conveyor belt 11 that is located underneath the compartment 5.

After the portion 23 has reached the area of effect of the heater 19, which corresponds to the illuminated portion of the conveyor belt 11, the portion 23 is heated to the process temperature due to the exposure of the portion 23 to the infrared light from the light source 6. Several thermometers 4, 7, and 8 are arranged in the compartment 5 to measure the actual temperature at several locations along the transport direction T. In some embodiments, the temperature can be precisely adjusted in the range of 30 degrees Celsius and 300 degrees Celsius.

When heated to the process temperature, the particles of the component of the substance in the powder portion 23 can melt and penetrate into the pores of the particles of the first excipient. In particular, the active pharmaceutical ingredient might melt and penetrate into the pores of the first excipient or the second excipient might melt and penetrate towards the pores of the first excipient, thereby causing the nanosized particles of the active pharmaceutical ingredient to penetrate into the pores of the first excipient.

The cooling can be carried out by use of a cooler 25 which comprises a housing or cooling compartment 9 arranged above the conveyor belt 11. The cooling compartment 9 houses a cooling element 10 which cools the portion 23 after it has been transported to the area of effect of the cooler 25 underneath the cooling element 10.

After cooling, the portion 23 of the generated chemical product can be delivered by the conveyor belt 11 onto a discharging element 12 which guides the portion 23 into a product collector 23 that can be a basket or the like.

In some embodiments, the conveyor belt 11 is operated at a constant velocity. In other embodiments, the conveyor belt 11 is operated intermittently.

The transport velocity of the conveyor belt 11 can be adjusted to the residence time of the powder portion 23 under the heater 5 such as to allow a penetration of the nanosized particles of the active pharmaceutical ingredient of the melt into the pores of the porous excipient. The time when the substance, such as the active pharmaceutical ingredient, is in the form of a melt ranges from a few seconds to tens of minutes.

The penetration of the melt of a pure active pharmaceutical ingredient or of a mixture with a second excipient into the pores of a porous first excipient is possible when melt wetts the walls of the capillaries, and when the viscosity of the melt is low enough.

The particles of the powder 23 that travel on the conveyor belt 11 under the infrared heater 19 can be heated, for example, to a temperature in the range of 30 and 300 degrees Celsius, preferably in the range of 40 and 250 degrees Celsius in steps of one degree Celsius.
A thermometer 4, 7, 8 can measure the temperature, for example, at the height of the powder portion 23 and the thermometer can be employed to control the heating to the desired process temperature.

The pharmaceutical first excipient which is in the manufacture of solid dispersions not melted and is porous, preferred mesoporous, can contain pores in the interval of 2 nm and 50 nm and can have a specific surface area between 200 m²/g and 1000 m²/g.

### LIST OF REFERENCE SIGNS

- 1: rotating roller
- 2: dispensing device
- 3: compaction element
- 4: thermometer
- 5: heating compartment, housing
- 6: infrared light source
- 7: thermometer
- 8: thermometer
- 9: cooling compartment, housing
- 10: cooling element
- 11: conveyor belt
- 12: product discharging element
- 13: product collector
- 15: transport device
- 17: support surface
- 19: heater
- 21: compaction device
- 23: portion of powder
- 25: cooler
- T: transport direction

## Claims

1. A method for producing a chemical product, in particular a pharmaceutical drug, the method comprising the steps of
providing a powder (23) comprising a porous first excipient and a substance, the substance comprising at least one component having a melting point which is below the melting point of the first excipient, and
producing the chemical product by heating the powder (23), in particular by use of an infrared light source, to a process temperature between the melting point of the component and the melting point of the first excipient.

2. The method of claim 1,
**characterized in that**
the substance comprises or consists of an active pharmaceutical ingredient which is the component that has the melting point below the melting point of the first excipient.

3. The method of claim 1,
**characterized in that**
the substance comprises an active pharmaceutical ingredient and a second excipient, the second excipient being the component having the melting point which is below the melting point of the first excipient.

4. The method of any one of claims 1 to 3,
**characterized in that**
the powder (23) is heated to the process temperature until the particles of the active pharmaceutical ingredient in the melted component have penetrated into the pores of the first excipient.

5. The method of any one of claims 1 to 4,
**characterized in that**
the heating step comprises exposing the powder (23) to infrared light provided by an infrared light source.

6. The method of any one of claims 1 to 5,
**characterized in that**
the heating step comprises moving the powder (23) along a transport direction (T), in particular while exposing the powder (23) to infrared light.

7. The method of any one of the preceding claims,
**characterized in that**
the powder (23) is compacted, in particular by use of a compaction device (21).

8. An apparatus for producing a chemical product, in particular a pharmaceutical drug, the apparatus comprising:
a heater (19) for generating the chemical product by heating a powder (23) when the powder (23) is arranged in an area of effect of the heater (19), the powder (23) comprising a porous first excipient and a substance, the substance comprising at least one component having a melting point which is below the melting point of the first excipient,
wherein the heater (19) comprises an infrared light source which is configured to heat the powder (23) to a process temperature between the melting point of the component and the melting point of the first excipient.

9. The apparatus of claim 8,
**characterized in that**
the apparatus comprises a transport device (15) for transporting the powder in a transport direction (T) to the heater (19) and/or for transporting the chemical product away from the heater (19), wherein, preferably the transport device (15) includes a conveyer belt (11) providing a support surface (17) for the powder (23) and/or the chemical product.

10. An apparatus for producing a chemical product,
in particular an apparatus in accordance with claim 8 or 9, the apparatus comprising:
a heater (19) for generating the chemical product by heating a powder (23) when the powder is arranged in an area of effect of the heater (19), the powder (23) comprising a porous first excipient and a substance, the substance comprising at least one component having a melting point which is below the melting point of the first excipient, and
a transport device (15) which comprises a conveyer belt (11) providing a support surface (17) for the powder (23) and/or the chemical product, the con veyor belt (11) being configured to move the powder (23) through the area of effect of the heater (19).

11. The apparatus of claim 9 or 10,
**characterized in that**
the heater (19) comprises an infrared light source (6) which is arranged, preferably in a housing (5), such as to illuminate a portion of the conveyor belt (11).

12. The apparatus of any one of the claims 8 to 11,
**characterized in that**
the heater (19) comprises at least one temperature sensor (4, 7, 8), and/or
the apparatus comprises a dispensing device (2) for providing the powder (23) on a transport device (15), in particular on a conveyor belt (11), which is configured to transport the powder (23) along a transport direction (T) to the heater (19).

13. The apparatus of any one of the claims 8 to 12,
**characterized in that**
the apparatus comprises a compaction device (21) for compacting the powder (23), wherein, preferably, the compaction device (21) comprises a compaction element (3) arranged above a conveyor belt (11) for transporting the powder (23) to the heater (19), wherein, further preferably, the compaction element (3) is movable towards the conveyor belt (11) for compression of the powder (23),
and/or wherein, preferably, the compaction device (21) is arranged between a dispensing device (2) for providing the powder (23) on a transport device (15) and the heater (15).

14. The apparatus of any one of the claims 8 to 13,
**characterized in that**
the apparatus comprises a cooler (9) for cooling the chemical product after removal from the heater (19), wherein, preferably, a transport device (15) is configured to transport the chemical product from the heater (19) to the cooler (9) and/or for removing the chemical product from the cooler (9).

15. Use of an apparatus in accordance with any one of the claims 8 to 14 to produce the chemical product.
